# FASCICULE DE BREVET EUROPEEN

(11) **EP 3 630 057 B1**
(45) Date de publication et mention de la délivrance du brevet: **18.08.2021**
(21) Numéro de dépôt: 18730991.9
(22) Date de dépôt: 31.05.2018
(51) Int. Cl.: A61K 8/46, A61K 8/60, A61Q 13/00, A61K 8/06

(54) **PARFUMS SOUS FORME DE MICROÉMULSIONS AQUEUSES**
DUFTSTOFFE IN FORM VON WÄSSRIGEN MIKROEMULSIONEN
PERFUMES IN THE FORM OF AQUEOUS MICROEMULSIONS

(30) Priorité: 01.06.2017 FR 1754850
(43) Date de publication de la demande: 08.04.2020
(73) Titulaire: Chanel Parfums Beauté, 92200 Neuilly-sur-Seine (FR); Université de Lille, 59000 Lille (FR)
(72) Inventeur: BOUCENNA VERDIER, Oriana, 93320 Les Pavillons Sous Bois (FR); RATAJ-NARDELLO, Véronique, 59710 Pont A Marcq (FR); AUBRY, Jean-Marie, 62590 Oignies (FR); DOUYERE, Grégory, 77250 Veneux-Les-Sablons (FR); MAINGUY, Corentine, 56000 Vannes (FR)
(74) Mandataire: Plasseraud IP
(86) Numéro de dépôt international: PCT/EP2018/064389
(87) Numéro de publication internationale: WO 2018/220147

(56) Documents cités:
- EP-A1- 2 340 804
- WO-A1-2014/090959
- FR-A1- 2 998 476
- J. I. GARCÍA ET AL: "Ecotoxicity studies of glycerol ethers in Vibrio fischeri : checking the environmental impact of glycerol-derived solvents", GREEN CHEMISTRY, vol. 17, no. 8, 1 janvier 2015 (2015-01-01), pages 4326-4333, XP055416779, GB ISSN: 1463-9262, DOI: 10.1039/C5GC00857C
- JOSÉ I. GARCÍA ET AL: "Green solvents from glycerol. Synthesis and physico-chemical properties of alkyl glycerol ethers", GREEN CHEMISTRY, vol. 12, no. 3, 1 janvier 2010 (2010-01-01), page 426, XP055025451, ISSN: 1463-9262, DOI: 10.1039/b923631g

## Description

L'invention concerne des microémulsions aqueuses volatiles parfumantes basées sur l'utilisation de solvo-surfactifs.

Classiquement, les parfums comprennent comme solvants des alcools, tels que l'éthanol ou encore l'isopropanol. L'utilisation de ces solvants présente, cependant, un certain nombre d'inconvénients : ils sont très volatils et inflammables, ce qui entraîne une certaine dangerosité lors de leur production, et dans une certaine mesure, de leur utilisation. D'autre part, leur propre odeur peut interférer avec celle du parfum. En outre, appliqués sur la peau ou les cheveux, ces parfums peuvent entraîner un dessèchement, en particulier chez les consommateurs à peau sensible.

On assiste donc actuellement, pour des raisons notamment de santé publique et/ou d'écologie, à l'émergence de nouvelles compositions parfumantes. L'objectif poursuivi est la diminution voire l'élimination des composés organiques volatils (tels que les alcools) contenus dans les parfums, en développant des compositions parfumantes sous forme de solutions ou de dispersions aqueuses stables.

Cependant, la plupart des molécules parfumantes sont hydrophobes et ne sont donc pas solubles dans l'eau. Pour contourner ce problème, il est connu d'utiliser des tensioactifs permettant de solubiliser les molécules parfumantes à l'intérieur de micelles formant des microémulsions. Il est souhaitable que les micelles gonflées contenant les parfums soient de petite taille pour que la composition parfumante ait un aspect transparent ou tout au moins translucide. Ainsi, la production de microémulsions répondant à ce critère d'aspect transparent revêt un intérêt majeur. Le document WO2014/090959 divulgue des microémulsions parfumantes de type huile dans-eau.

D'autres contraintes sont liées à la stabilité thermodynamique de la microémulsion, au caractère non-collant de celle-ci et à l'absence de résidu sur la peau ou sur les vêtements. Il est donc important de pouvoir les préparer en utilisant le moins de tensioactif possible.

Il existe donc un besoin pour une composition parfumante stable, contenant une quantité importante de parfum, qui soit transparente ou tout au moins translucide, et qui contienne le moins de tensioactif possible.

La présente invention a pour but de fournir des microémulsions aqueuses, transparentes, substantiellement exemptes d'éthanol, contenant au moins une substance hydrophobe parfumante (de préférence au moins 3% et préférentiellement environ 10% de parfum), et au moins un solvo-surfactif volatil. De telles microémulsions odoriférantes ou parfumantes sont stables, et comprennent le moins possible de substances provoquant des effets indésirables, en particulier sur la peau et/ou sur l'environnement.

Selon l'invention, une substance est « volatile » lorsque sa température d'ébullition est inférieure à 250°C à pression atmosphérique. Les composés « non volatils » ont une température d'ébullition supérieure à 250°C à pression atmosphérique.

La présente invention se rapporte donc à une microémulsion de type huile-dans-eau comprenant, de préférence consistant en, en poids par rapport au poids total de microémulsion :
* 70% à 94%, de préférence 70% à 90%, d'eau
• 1% à 15%, de préférence 5% à 12%, d'au moins une substance hydrophobe parfumante,
• 4% à 20%, de préférence 4% à 18%, d'au moins un solvo-surfactif, de préférence volatil, qui est un dérivé de glycérol monoalkylé de formule (I) suivante : 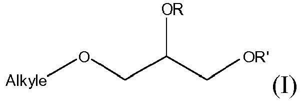 dans laquelle le groupe « Alkyle » est un groupe alkyle linéaire comprenant 5 atomes de carbone, et R et R' sont chacun indépendamment H ou un groupe alkyle linéaire ou ramifié comprenant de 1 à 5 atomes de carbone, de préférence un groupe méthyle ou éthyle, à la condition que R est différent de R'.
• 0,1% à 15%, de préférence 1% à 13%, d'au moins un tensioactif anionique et d'au moins un alkylglucoside en tant qu'agent hydrotrope.

Par « microémulsion de type huile-dans-eau », on désigne un système liquide dans lequel une phase huileuse (ou hydrophobe) est dispersée dans une phase continue aqueuse (ou hydrophile), de sorte à former des gouttes de diamètre inférieur à 100 nm. L'interface huile/eau est stabilisée par des composés tensioactifs. De préférence, les gouttes ont un diamètre compris entre 2 et 100 nm.

Ces microémulsions présentent des gouttes qui sont invisibles à l'œil nu et au microscope optique. Elles sont transparentes, tout au moins translucides, contrairement aux émulsions, ce qui est une propriété recherchée notamment pour les compositions parfumantes.

Par « substance hydrophobe », on désigne un corps pur ou un mélange insoluble ou très peu soluble dans l'eau, par nature. Une méthode possible pour déterminer l'hydrophobicité des substances est de mesurer leur solubilité dans différents solvants ou le temps de rétention sur colonne chromatographique (par chromatographie en phase liquide à haute performance HPLC) de ladite substance hydrophobe.

Les substances hydrophobes selon l'invention sont parfumantes, i.e. elles sont odoriférantes et peuvent être utilisées dans les parfums. Par « substance odoriférante » on entend une substance détectable olfactivement par un sujet et/ou par olfactométrie, selon des principes connus de l'homme du métier. Un exemple de méthode permettant la détection d'une substance odoriférante est décrit dans le document EP 0003088. D'autres techniques de détection d'une substance odoriférante sont applicables comme les techniques de chromatographie en phase gazeuse, de spectroscopie de masse ou encore d'analyse par absorption infrarouge. On entend aussi par substance odoriférante une substance qui répand une odeur, de préférence agréable pour au moins 20% de personnes, en particulier un parfum.

De préférence, la substance hydrophobe parfumante est une substance hydrophobe parfumante naturelle ou synthétique, plus préférentiellement naturelle. Plus préférentiellement, elle est choisie parmi les terpènes, les huiles essentielles et les composés naturels présentant des propriétés odoriférantes (terpénoïdes), notamment choisis parmi les aldéhydes, les esters, les cétones, les alcools, les phénols, les alcènes et les éthers.

Par "terpènes", on désigne des hydrocarbures dont l'élément de base est l'isoprène, leur formule brute comportant un nombre de carbones multiple de 5, en particulier des terpènes contenant notamment 10 ou 15 atomes de carbone, utilisés en parfumerie.

Par "terpénoïdes", on désigne des dérivés des terpènes, par exemple des alcools, des phénols, des cétones, des aldéhydes, des esters ou des éthers.

Terpènes et terpénoïdes sont contenus dans les "huiles essentielles", désignant le liquide concentré, très souvent odoriférant, volatil, produit par les plantes. Les huiles essentielles sont le plus souvent extraites des organes de la plante par hydrodistillation notamment, mais les constituants de ces huiles sont largement synthétisés par l'industrie.

On peut notamment utiliser les substances hydrophobes parfumantes naturelles suivantes :
- terpènes: pinènes, camphènes, limonène, cadinène, carène, caryophyllène,
- alcools : linalol, géraniol, menthol, citronellol,
- cétones: menthone, carvone, béta-ionone, thuyone, camphre, cyclopentadécanone,
- aldéhydes : citral, citrannal, citronellal, aldéhyde cinnamique, lilial,
- esters: acétate de linalyle, acétate de menthyle, acétate de géranyle, succinate de géranyle, phénols : thymol, carvacrol, eugénol, isoeugénol,
- éthers: anéthol, eucalyptol, cinéol, oxyde de rose,
- alcènes : limonène.

Les huiles essentielles peuvent être les huiles d'ylang-ylang, de bergamote, d'eucalyptus, de lavande, de lavandin, de lemongrass, de patchouli, de menthe poivrée, de pin, de rose, de coriandre, de Shiu, de sauge, de géranium, de palmarosa, de LitseaCubeba, de citron, de citronnelle, de fleur d'oranger, de pamplemousse, de lime, de mandarine, de tangerine, d'orange, de cajeput, de camphre, de romarin, d'anis vert, d'anis étoilé, de fenouil, de basilic, d'estragon, de girofle, de piment, de thym, de sassafras, d'absinthe, d'armoise, de cèdre, d'hysope, de tagetes, de rue, d'élémi, de galbanum, de baies de genièvre, de cabreuva, de bois de gaiac, de bois de santal, de vétiver, d'ambrette, d'angélique, de rhizome d'iris, de carotte, de céleri, de cumin, de livèche, de persil, de cannelle, de cardamome, de gingembre, de noix de muscade, de poivre, d'oliban, de myrrhe, de baume du Pérou, de styrax, de buchu, de camomille ou de ciste (Jean Garnero, "Huiles essentielles", Techniques de l'ingénieur, Traité constantes physico-chimiques, K-345).

La quantité de substance hydrophobe parfumante dans les microémulsions de l'invention est de 1% à 15% en poids, de préférence de 5% à 12% en poids par rapport au poids total de microémulsion.

Par « solvo-surfactif », on désigne un composé amphiphile, cumulant certaines propriétés des tensioactifs, notamment la diminution des tensions superficielle eau/air et interfaciales huile/eau, la capacité d'auto-association dans l'eau, et certaines propriétés des solvants, notamment la capacité à s'évaporer sans laisser de résidus.

D'une façon surprenante la Demanderesse a découvert et démontré que l'utilisation d'un solvo-surfactif de formule (I) tel que défini dans la revendication 1 permettait d'obtenir une composition parfumante stable, qui est transparente ou tout au moins translucide, qui comprend une quantité importante de parfum et le moins de tensioactif possible.

Le solvo-surfactif présent dans la microémulsion de l'invention est le dérivé de glycérol monoalkylé de formule (I) telle que définie ci-dessus, à savoir : 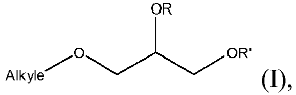 dans laquelle le groupe « Alkyle » est un groupe alkyle linéaire comprenant 5 atomes de carbone, et R et R' sont chacun indépendamment H ou un groupe méthyle ou éthyle, à la condition que R est différent de R'.

Le dérivé de glycérol monoalkylé de formule (I) dans laquelle le groupe « Alkyle » est un groupe alkyle linéaire comprenant 3 atomes de carbone (groupe propyle), R est H et R' est méthyle, est le 1-méthoxy-3-propoxypropan-2-ol. Il est appelé « C301 » dans les exemples de la présente demande.

Le dérivé de glycérol monoalkylé de formule (I) dans laquelle le groupe « Alkyle » est un groupe alkyle linéaire comprenant 3 atomes de carbone (groupe propyle), R est méthyle et R' est H, est le 2-méthoxy-3-propoxypropan-1-ol. Il est appelé « C310 » dans les exemples de la présente demande.

Le dérivé de glycérol monoalkylé de formule (I) dans laquelle le groupe « Alkyle » est un groupe alkyle linéaire comprenant 4 atomes de carbone (groupe butyle), R est H et R' est méthyle, est le 1-méthoxy-3-butoxypropan-2-ol. Il est appelé « C401 » dans les exemples de la présente demande.

Le dérivé de glycérol monoalkylé de formule (I) dans laquelle le groupe « Alkyle » est un groupe alkyle linéaire comprenant 4 atomes de carbone (groupe butyle), R est méthyle et R' est H, est le 2-méthoxy-3-butoxypropan-1-ol. Il est appelé « C410 » dans les exemples de la présente demande.

Le dérivé de glycérol monoalkylé de formule (I) dans laquelle le groupe « Alkyle » est un groupe alkyle linéaire comprenant 5 atomes de carbone (groupe pentyle), R est H et R' est méthyle, est le 1-méthoxy-3-pentoxypropan-2-ol. Il est appelé « C501 » dans les exemples de la présente demande.

Le dérivé de glycérol monoalkylé de formule (I) dans laquelle le groupe « Alkyle » est un groupe alkyle linéaire comprenant 5 atomes de carbone (groupe pentyle), R est méthyle et R' est H, est le 2-méthoxy-3-pentoxypropan-1-ol. Il est appelé « C510 » dans les exemples de la présente demande.

Plus préférentiellement, le solvo-surfactif présent dans la microémulsion de l'invention est le dérivé de glycérol monoalkylé de formule (I) tel que défini ci-dessus dans laquelle le groupe « Alkyle » est un groupe alkyle linéaire comprenant 5 atomes de carbone. Ce composé est le 2-méthoxy-3-pentoxypropan-1-ol, appelé « C510 ».

La quantité de solvo-surfactif volatil dans la microémulsion de l'invention est de 4% à 20% en poids, de préférence de 4% à 18% en poids, et encore plus préférentiellement de 4% à 10% en poids.

Par « tensioactif », on désigne un composé de nature amphiphile, non volatil, comportant une partie hydrophile et polaire, et une partie hydrophobe apolaire. Un tensioactif abaisse la tension superficielle des solutions aqueuses et diminue la tension interfaciale entre l'eau et un liquide organique non miscible. Il permet ainsi de solubiliser deux phases non miscibles, telles que l'eau et l'huile, en interagissant par sa partie polaire avec l'eau et par sa partie apolaire avec l'huile.

Le tensioactif présent dans la microémulsion selon l'invention est un tensioactif anionique, à savoir un tensioactif dont la partie hydrophile est chargée négativement. Les tensioactifs anioniques se sont montrés les plus efficaces, dans le cadre de l'invention, par rapport aux tensioactifs non-ioniques, amphotères, ou cationiques. Par plus efficace, il faut entendre au sens de l'invention, une introduction de tensioactif en quantité plus faible pour former une microémulsion.

Selon un mode de réalisation avantageux, le tensioactif anionique présent dans la microémulsion de l'invention est choisi parmi :
- les alkylsulfonates, et notamment :
   - le sodium C14-17 sulfonate (SAS),
   - le sulfosuccinate de dihexyle (DHS) de formule : 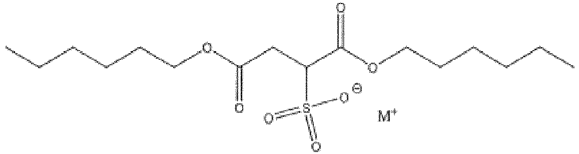 dans laquelle M⁺ représente Na⁺, K⁺, NH₄⁺, (HOCH₂CH₂)₃NH⁺, ou
   - le sulfosuccinate d'éthyl-2-hexyle (Aérosol OT® ou AOT de chez CYTEC) de formule : 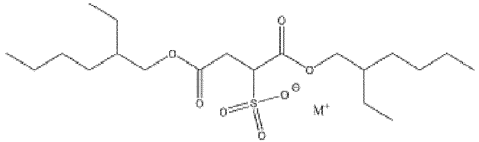 dans laquelle M⁺ représente Na⁺, K⁺, NH₄⁺, (HOCH₂CH₂)₃NH⁺,
- les alkylarylsulfonates de formule : 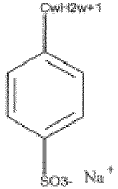 dans laquelle w est un entier compris de 8 à 12,
   et notamment l'isooctylbenzènesulfonate de sodium, l'isononyl benzènesulfonate de sodium ou l'isododécylbenzènesulfonate de sodium de formule : 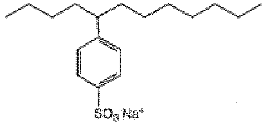
- les propoxysulfates de formule : 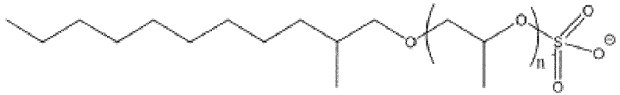 dans laquelle le nombre de motifs propoxylate n est compris de 4 à 8,
- les alkylsulfates, notamment les sels du sulfate de lauryle tel que le lauryl sulfate de sodium encore dénommé dodécyl sulfate de sodium (SDS), le lauryl sulfate d'ammonium (ALS) ; les alkyléther sulfates de sodium tel que le lauryléther (laureth) sulfate de sodium (LES) ; le sodium coco sulfate (SCS),
- et les sels d'acides gras de formule R-CO2⁻ M⁺, dans laquelle R représente une chaîne carbonée, linéaire ou ramifiée, saturée ou insaturée, contenant 8 à 18 atomes de carbone, et M⁺ représente un cation choisi parmi les ions Na⁺, K⁺, NH₄⁺, (HOCH₂CH₂)₃NH⁺, notamment le sel d'acide oléique de formule CH₃(CH2)₇CH=CH(CH₂)₇CO₂⁻ M⁺ dans lequel M⁺ a les significations définies ci-dessus.

La microémulsion comprend un alkyl glucoside en tant qu'agent hydrotrope. Par « agent hydrotrope », on entend un composé amphiphile, comprenant des groupes fonctionnels hydrophiles, utilisé pour permettre la solubilisation de substances faiblement solubles dans une solution aqueuse.

D'une façon surprenante, la Demanderesse a démontré que la combinaison d'au moins un tensioactif anionique et d'au moins un alkylglucoside permet d'augmenter le point trouble de la microémulsion.

Par point trouble selon l'invention, on entend la température à partir de laquelle la microémulsion passe d'un état transparent (voire translucide) à un état trouble voire laiteux. La microémulsion selon l'invention doit garder une stabilité dans des conditions d'usages classiques et de fait doit être stable, et donc rester transparente voire translucide, dans une gamme de températures comprises entre 5°C et 45°C incluses.

Ainsi grâce à une combinaison judicieuse de tensioactif et d'alkylglucoside il est possible d'obtenir une microémulsion stable en utilisant une très faible quantité de tensioactif. Des microémulsions ne contenant que le tensioactif seul, ou ne contenant que l'alkylglucoside seul, à des quantités équivalentes ou quasi-équivalentes ne sont en revanche pas stables. Il existe donc un effet de synergie entre le tensioactif anionique et l'alkylglucoside.

En outre, cet effet de synergie a également un impact positif sur la quantité de solvo-surfactif introduite dans la microémulsion. Ainsi grâce à l'utilisation conjointe de tensioactif anionique et d'alkylglucoside, il est possible d'utiliser des quantités de solvosurfactif plus faibles, à savoir allant de de 4% à 10% en poids par rapport au poids total de la microémulsion. Dans les exemples ci-après, une quantité de 5% de solvo-surfactif permet d'obtenir une microémulsion stable et une parfaite solubilisation de la substance hydrophobe parfumante. La diminution de la quantité de solvo-surfactif se fait au profit de la quantité d'eau qui peut ainsi être augmentée. Le caractère aqueux de la microémulsion est ainsi augmenté.

Selon un mode de réalisation avantageux, l'alkyl glucoside présent dans la microémulsion de l'invention est choisi parmi l'heptyl glucoside, l'octyl glucoside, le décyl glucoside et leurs mélanges, et est de préférence l'heptyl glucoside.

La quantité de tensioactif anionique et d'alkylglucoside présents dans la microémulsion de l'invention est de 0,1% à 15% en poids, de préférence de 1% à 13% en poids par rapport au poids total de microémulsion.

Plus particulièrement, dans la microémulsion de l'invention :
- la quantité de tensioactif anionique est inférieure ou égale à 2% en poids par rapport au poids total de microémulsion,
- la quantité d'alkylglucoside est de 0,5% à 13% en poids par rapport au poids total de microémulsion, et de préférence de 0,5% à 8%.

Selon un mode de réalisation avantageux de l'invention, le ratio tensioactif anionique : alkylglucoside est de 1 : 4 à 1 : 12, et de préférence de 1 :4 à 1 :10.

De préférence, la microémulsion selon l'invention consiste en :
- 70% à 90% d'eau,
- 5% à 12% d'au moins une substance hydrophobe parfumante,
- 4% à 18% d'au moins un solvo-surfactif volatil,
- 1% à 13%, d'au moins un tensioactif anionique et d'au moins un alkylglucoside en tant qu'agent hydrotrope.

De préférence, la microémulsion selon l'invention est substantiellement exempte d'éthanol, i.e. elle comprend moins de 3% en poids d'éthanol, de préférence moins de 2% en poids d'éthanol, de préférence moins de 1% en poids d'éthanol. Plus préférentiellement, elle est dépourvue d'éthanol, i.e. elle contient 0% en poids d'éthanol.

La microémulsion selon l'invention permet notamment d'obtenir une composition parfumante aqueuse présentant une transparence similaire à un produit alcoolique, sans toucher gras ou collant, et ne laissant pas de traces ou de résidus sur les tissus ou la peau.

Optionnellement, la microémulsion selon l'invention peut comprendre de 0,01% à 2% en poids d'au moins un additif.

Selon un mode de réalisation préféré, la microémulsion selon l'invention peut comprendre, de manière non limitative, un ou plusieurs des additifs suivants:
- un agent anti-mousse choisi parmi le décaméthyl cyclopentasiloxane, la diméthicone, le cétyl diméthicone, le diméhicone copolyol, le diméthiconol, l'hexaméthyldisiloxane, l'hexamidine diiséthionate, l'hexyl alcohol, l'hexyldeceth-2, l'isopropyl alcohol, le phénethyl disiloxane, le phényl triméthicone, le polysilicone-10, le polysilicone-7, le polysilicone-8, le propyl alcohol, le silica diméthyl silylate, le silica silylate, le siméthicone, le tetraméthyl decynediol et le triméthylsiloxysilicate;
- un conservateur choisi par exemple parmi le sodium benzoate, le potassium benzoate, le sodium salicylate, le caprylyl glycol, le pentylène glycol, l'éthylhéxylglycerine, le chlorphénesine, la chlorhexidine, le chlorhexidine digluconate, le méthylisothiazolinone, le méthylparaben, le propylparaben, le phénéthyl alcohol et le phénoxyethanol;
- un antioxydant choisi par exemple parmi le BHA, le BHT et le tocophérol.

Les microémulsions selon l'invention ont une qualité de fragrance stable dans le temps correspondant à la durée de vie standard d'un produit cosmétique, et stable en température de 5 à 45°C, ce qui correspond aux températures d'exposition et d'usage d'un produit cosmétique.

Les critères techniques de la qualité d'une fragrance, dans le cas d'une fragrance aqueuse, sont :
- la capacité d'une composition parfumée à maintenir, après application, un seuil de perception olfactif dans le temps,
- la capacité d'une composition à maintenir, une fois appliquée, sa forme olfactive dans le temps,
- la capacité d'une composition à ne pas subir d'altérations endogènes ou exogènes, qui pourraient modifier sa forme olfactive, et
- l'innocuité, qui est la faculté d'une composition à ne pas produire d'effets indésirables une fois appliquée sur la peau de l'utilisateur.

De façon très avantageuse, les microémulsions de l'invention sont stables thermodynamiquement et ont un aspect transparent ou translucide et de façon encore plus avantageuse, les microémulsions de l'invention sont stables thermodynamiquement et ont un aspect transparent ou translucide pendant au moins un ou deux ans.

Les microémulsions de l'invention sont avantageusement utilisées pour la préparation de compositions appliquées à :
- la parfumerie fine, ou
- la cosmétique et les produits d'hygiène corporelle.

Ainsi, la présente invention se rapporte également à l'utilisation d'une microémulsion selon l'invention, pour la préparation d'une composition de parfumerie fine, ou d'une composition cosmétique ou d'hygiène corporelle.

Les microémulsions peuvent être utilisées en cosmétique. Elles peuvent alors contenir notamment, et sans que cette liste soit limitative, un ou plusieurs des composés choisis parmi des silicones, de l'huile paraffine, de l'isooctane, de l'isodécane, du squalène, du squalane, du sébum et de la lanoline.

Dans les exemples qui suivent, les noms, abréviations et structures des solvo-surfactifs testés, à savoir les dérivés de glycérol monométhylés (I), sont les suivants :

| NOMS | ABREVIATIONS | STRUCTURES |
|---|---|---|
| Propylglycérolmonométhylé | C301 | 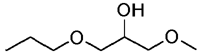 |
| Propylglycérolmonométhylé | C310 | 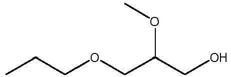 |
| Butylglycérolmonométhylé | C401 | 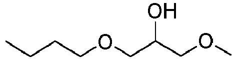 |
| Butylglycérolmonométhylé | C410 | 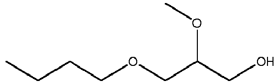 |
| Pentylglycérolmonométhylé | C501 | 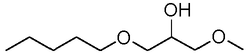 |
| Pentylglycérolmonométhylé | C510 | 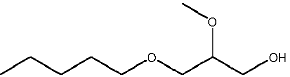 |

### Exemple 1 : Synthèse des solvo-surfactifs selon l'invention, les dérivés de glycérol monométhylés de formule (I)

Les synthèses du 1-méthoxy-3-propoxypropan-2-ol (C301) et du 1-méthoxy-3-pentoxypropan-2-ol (C501) se réalisent en deux étapes. La synthèse du 1-méthoxy-3-butoxypropan-2-ol (C401) est réalisée simplement par ouverture de l'époxyde dans la mesure où le réactif de départ, le butylglycidol, est accessible commercialement.

### a) Condensation de l'alcool sur l'épichlorhydrine

La condensation de l'alcool (1 mole) sur l'épichlorhydrine se fait en léger excès d'épichlorhydrine (1,5 moles) en présence de ZnCl₂ comme catalyseur. L'épichlorhydrine est ajoutée goutte à goutte pendant 1h à 100°C. Le milieu réactionnel est ensuite maintenu à 115°C pendant 5h puis refroidi à 50°C. Un ajout goutte à goutte pendant 1h de NaOH (2,3 moles) à 48% est alors effectué. Toutes ces étapes de synthèse sont réalisées sous forte agitation. Une fois la réaction terminée, on ajoute de l'eau distillée et le produit est ainsi lavé 2 fois à l'eau pour éliminer les sels résiduels.

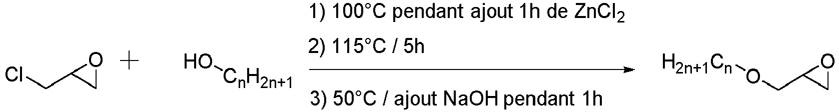

### Synthèse du Cₙglycidol, où n = 3,4,5

Le produit obtenu est enfin distillé sous vide à 10-20 mbars entre 75 et 80°C.

### b) Ouverture de l'époxyde avec le méthanolate

Le Cₙglycidol (où n = 3, 4, 5) est ajouté goutte à goutte à une solution de méthanol contenant du méthanolate de sodium préalablement obtenu par réaction entre le méthanol et du sodium solide. L'ajout est effectué à reflux à 80°C pendant 30 min, puis la température de 80°C est maintenue pendant 24h jusqu'à disparition totale de Cₙglycidol :

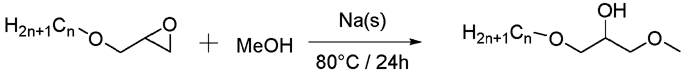

### Ouverture de l'époxyde par du méthanolate de sodium

### Purification :

Le méthanol est évaporé à l'évaporateur rotatif une fois la réaction terminée. Le produit obtenu est ensuite lavé avec deux solutions aqueuses saturées en NaCl: l'une contenant 3,4% de HCl et l'autre contenant 10% de NaHVCO₃.

### c) Synthèse des composés C310, C410, et C510

La synthèse des composés C310, C410 et C510 est réalisée en trois étapes à partir du Cₙglycidol (où n = 3, 4, 5) telle que décrite dans le schéma 1 ci-après.

La première étape s'effectue dans les conditions de l'étape b) précédemment décrite, en remplaçant le méthanol par de l'alcool benzylique. Dans une seconde étape, l'intermédiaire ainsi obtenu est mis à réagir en milieu basique en présence de diméthylsulfate afin d'obtenir la méthylation de la fonction alcool secondaire libre. Après une troisième étape d'hydrogénation catalytique en présence d'hydrogène et de palladium sur charbon, le produit final est obtenu.

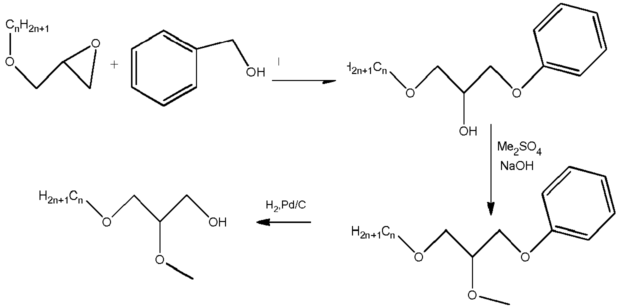

### Synthèse des solvo-surfactifs C310, C410 et C510

### Exemple 2 : Solubilisation de concentrés de parfums

### Solubilisation par les solvo-surfactifs seuls

### Solubilisation du parfum gamma-undecalactone

Le but de cet essai est de classer les solvo-surfactifs selon leur capacité à solubiliser (ou non) le gamma-undecalactone.

Des formules contenant 5% de gamma-undecalactone, 4,4% de SDS (tensioactif anionique), X% de solvo-surfactant et QSP sur l'eau ont été réalisées.

Le tableau ci-après détaille la quantité de SS nécessaire pour solubiliser le parfum.

| C510 | C501 | C401 | C410 | C310 |
|---|---|---|---|---|
| 9,1% | 9,3% | 12,2% | 16,2% | 20% |

On obtient le classement suivant: C510 > C501 > C401 > C410 > C310.

Le solvo-surfactif présentant les meilleures capacités à solubiliser la substance hydrophobe parfumante est le C510.

### Exemple 3 : Stabilité en température de microémulsions aqueuses parfumantes

Le jus de parfum suivant (P) a été préparé :

| Nom du parfum | CAS # | Quantité (%) |
|---|---|---|
| UNDECALACTONE GAMMA | 104-67-6 | 0,62 |
| CIS 3 HEXENYL ACETATE | 3681-71-8 | 1,21 |
| HEXENYLE CIS 3 BENZOATE | 25152-85-6 | 2,42 |
| EUGENOL | 97-53-0 | 2,42 |
| IONONE BETA | 14901-07-6 | 3,64 |
| METHYLIONONE GAMMA | 127-51-5 | 6,06 |
| BENZYL PROPIONATE | 122-63-4 | 1,21 |
| BENZYLE ACETATE | 140-11-4 | 6,06 |
| HEDIONE HC | 24851-98-7 | 36,36 |
| ISO GAMMA SUPER | 68155-66-8 | 31,52 |
| Cis 3 HEXENYLE SALICYLATE | 65405-77-8 | 6,06 |
| VANILINE | 121-33-5 | 2,42 |

Puis les formulations suivantes ont été préparées :

| Formulation | Eau | Parfum **(P)** | Solvo-surfactif **(C510)** | Tensioactif **(**SDS) | Alkylglucoside (**Heptyl-glucoside)** | Stabilité 5°C à 45°C |
|---|---|---|---|---|---|---|
| **A** | 80.4% | 5% | 12.5% | 2.1% | NON | KO |
| **B** | 78.9% | 5% | 15% | NON | 1.1% | KO |
| **C** | 76.7% | 5% | 15% | 2.0% | 1.3% | OK |
| **D** | 85.34% | 5% | 5% | 0.66% | 4.0% | OK |
| **E** | 85.0% | 5% | 5% | NON | 5.0% | KO |

Pour être acceptables, les formulations A, B, C, D et E doivent être stables en température sur un intervalle allant de 5°C à 45°C.

### Les formulations sont donc placées dans un bain à 5°C et à 45°C, pendant 24h.

Après observation, les formulations limpides sont considérées comme stables et les formulations troubles sont considérées instables. Le tableau ci-dessus reporte les compositions stables (« OK ») et les compositions ayant conduit à une déstabilisation (apparition d'un trouble) (« KO »).

Seules les formulations C et D comprenant une association d'un tensioactif anionique et d'un alkylglucoside présentent une stabilité sur l'ensemble de la plage de température 5°C à 45°C.

Avantageusement la composition D présente l'ensemble des bénéfices de l'invention grâce à la mise en œuvre d'un ratio judicieux « tensioactif : alkylglucoside » qui est de 1:6, permettant d'obtenir une formulation aqueuse riche en eau (> à 85%) en présence d'une faible quantité de solvo-surfactif (5%).

### Exemple 4 : Test comparatif

Une microémulsion de l'invention, à savoir la formulation D décrite dans l'exemple 3 précédent, qui comprend le solvo-surfactif C510, est comparée à deux autres microémulsions (formulations D1 et D2) qui comprennent respectivement de l'hexylène glycol et du propylène glycol à la place du solvo-surfactif C510.

L'hexylène glycol et le propylène glycol ne répondent pas à la formule (I) du solvo-surfactif de l'invention.

| Formulation | D (invention) | D1 | D2 |
|---|---|---|---|
| Eau | 85,34% | 85,34% | 85,34% |
| Parfum (P) | 5% | 5% | 5% |
| SDS | 0,66% | 0,66% | 0,66% |
| Heptyl glucoside | 4% | 4% | 4% |
| C510 | 5% | - | - |
| Hexylène glycol | - | 5% | - |
| Propylène glycol | - | - | 5% |

Le jus de parfum P est tel que décrit à l'exemple 3.

L'heptyl glucoside utilisé est celui commercialisé par Seppic sous la dénomination « Sepiclear G7 ».

Le lauryl sulfate de sodium (SDS) utilisé est celui commercialisé par BASF sous la dénomination « Texaplon LS30 »

Comme déjà indiqué, pour être acceptables, les formulations D, D1 et D2 doivent être stables en température sur un intervalle allant de 5°C à 45°C.

Les formulations sont placées dans un bain à 5°C et à 45°C, pendant 24h.

Après observation, les formulations limpides sont considérées comme stables et les formulations troubles sont considérées instables.

Il est ainsi observé qu'à J0, à température ambiante, la formulation D est limpide tandis que les formulations D1 et D2 sont déjà opalescentes.

Au bout de 24 heures, aussi bien à 5°C qu'à 45°C, les formulations D1 et D2 sont troubles et un déphasage important est observé, tandis que la formulation D de l'invention est restée limpide.

Ce test comparatif démontre donc l'importance jouée par le solvo-surfactif de l'invention.

## Revendications

1. Microémulsion de type huile-dans-eau comprenant, en poids par rapport au poids total de microémulsion :
• 70% à 94%, de préférence 70% à 90%, d'eau
• 1% à 15%, de préférence 5% à 12%, d'au moins une substance hydrophobe parfumante,
• 4% à 20%, de préférence 4% à 18%, d'au moins un solvo-surfactif, de préférence volatil, qui est un dérivé de glycérol monoalkylé de formule (I) suivante : 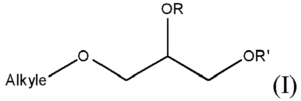 dans laquelle le groupe « Alkyle » est un groupe alkyle linéaire comprenant 5 atomes de carbone et R et R' sont chacun indépendamment H ou un groupe alkyle linéaire ou ramifié comprenant de 1 à 5 atomes de carbone, de préférence un groupe méthyle ou éthyle, à la condition que R est différent de R'.
• 0,1% à 15%, de préférence 1% à 13%, d'au moins un tensioactif anionique et d'au moins un alkylglucoside en tant qu'agent hydrotrope.

2. Microémulsion selon la revendication 1, dans laquelle la substance hydrophobe parfumante est une substance hydrophobe parfumante naturelle choisie parmi les terpènes, les huiles essentielles et les composés naturels présentant des propriétés odoriférantes, notamment choisis parmi les aldéhydes, les esters, les cétones, les alcools, les phénols, les alcènes et les éthers.

3. Microémulsion selon l'une quelconque des revendications 1 à 2, dans laquelle le tensioactif anionique est choisi parmi :
• les alkylsulfonates, et notamment :
- le sodium C14-17 sulfonate (SAS),
- le sulfosuccinate de dihexyle (DHS) de formule : 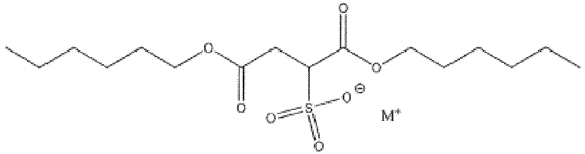 dans laquelle M⁺ représente Na⁺, K⁺, NH₄⁺, (HOCH₂CH₂)₃NH⁺, ou
- le sulfosuccinate d'éthyl-2-hexyle de formule : 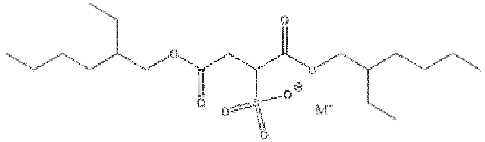 dans laquelle M⁺ représente Na⁺, K⁺, NH₄⁺, (HOCH₂CH₂)₃NH⁺,
• les alkylarylsulfonates de formule : 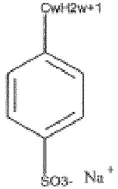 dans laquelle w est un entier compris de 8 à 12,
et notamment l'isooctylbenzènesulfonate de sodium, l'isononyl benzènesulfonate de sodium ou l'isododécylbenzènesulfonate de sodium de formule : 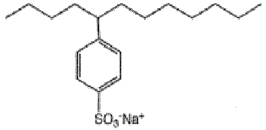
• les propoxysulfates de formule : 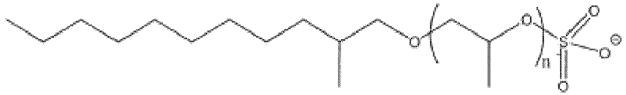 dans laquelle le nombre de motifs propoxylate n est compris de 4 à 8,
• les alkylsulfates, notamment les sels du sulfate de lauryle tel que le lauryl sulfate de sodium encore dénommé dodécyl sulfate de sodium (SDS), le lauryl sulfate d'ammonium (ALS) ; les alkyléther sulfates de sodium tel que le lauryléther (laureth) sulfate de sodium (LES) ; le sodium coco sulfate (SCS),
• et les sels d'acides gras de formule R-CO₂⁻ M⁺, dans laquelle R représente une chaîne carbonée, linéaire ou ramifiée, saturée ou insaturée, contenant 8 à 18 atomes de carbone, et M⁺ représente un cation choisi parmi les ions Na⁺, K⁺, NH₄⁺, (HOCH₂CH₂)₃NH⁺, notamment le sel d'acide oléique de formule CH₃(CH2)₇CH=CH(CH₂)₇CO₂⁻ M⁺ dans lequel M⁺ a les significations définies ci-dessus.

4. Microémulsion selon l'une quelconque des revendications 1 à 3, dans laquelle l'alkyl glucoside est notamment choisi parmi l'heptyl glucoside, l'octyl glucoside, le décyl glucoside et leurs mélanges, et est de préférence l'heptyl glucoside.

5. Microémulsion selon l'une quelconque des revendications 1 à 4, dans laquelle :
- la quantité de tensioactif anionique est inférieure ou égale à 2% en poids par rapport au poids total de microémulsion,
- la quantité d'alkylglucoside est de 0,5% à 13% en poids par rapport au poids total de microémulsion, et de préférence de 0,5% à 8%.

6. Microémulsion selon l'une quelconque des revendications 1 à 5, dans laquelle le ratio tensioactif anionique : alkylglucoside est de 1 : 4 à 1 : 12, et de préférence de 1 :4 à 1 :10.

7. Microémulsion selon l'une quelconque des revendications 1 à 6, dans laquelle la quantité de solvo-surfactif est de 4% à 10% en poids par rapport au poids total de microémulsion.

8. Microémulsion selon l'une quelconque des revendications 1 à 7, qui est substantiellement exempte d'éthanol, de préférence qui est dépourvue d'éthanol.

9. Utilisation d'une microémulsion selon l'une quelconque des revendications 1 à 8, pour la préparation d'une composition de parfumerie fine, ou d'une composition cosmétique ou d'hygiène corporelle.

## Patentansprüche

1. Mikroemulsion vom Öl-in-Wasser-Typ, umfassend, in Gewicht bezogen auf das Gesamtgewicht der Mikroemulsion:
• 70 % bis 94 %, vorzugsweise 70 % bis 90 %, Wasser
• 1 % bis 15 %, vorzugsweise 5 % bis 12 %, mindestens eines hydrophoben Duftstoffs
• 4 % bis 20 %, vorzugsweise 4 % bis 18 %, mindestens eines Lösungsmitteltensids, vorzugsweise flüchtig, das ein monoalkyliertes Glycerinderivat der folgenden Formel (I) ist: 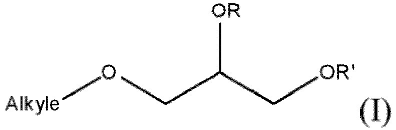 worin die "Alkyl"-Gruppe eine lineare Alkylgruppe ist, die 5 Kohlenstoffatome umfasst, und R und R' jeweils unabhängig voneinander H oder eine lineare oder verzweigte Alkylgruppe sind, die 1 bis 5 Kohlenstoffatome umfasst, vorzugsweise eine Methyl- oder Ethylgruppe, mit der Maßgabe, dass R verschieden von R' ist,
• 0,1 % bis 15 %, vorzugsweise 1 % bis 13 %, mindestens eines anionischen Tensids und mindestens eines Alkylglucosids als hydrotropes Mittel.

2. Mikroemulsion nach Anspruch 1, wobei der hydrophobe Duftstoff ein natürlicher hydrophober Duftstoff ist, ausgewählt aus Terpenen, ätherischen Ölen und natürlichen Verbindungen mit duftenden Eigenschaften, insbesondere ausgewählt aus Aldehyden, Estern, Ketonen, Alkoholen, Phenolen, Alkenen und Ethern.

3. Mikroemulsion nach Anspruch 1 oder 2, wobei das anionische Tensid ausgewählt ist aus:
• Alkylsulfonaten, und insbesondere:
- C14-17-Natriumsulfonat (SAS),
- Dihexylsulfosuccinat (DHS) der Formel: 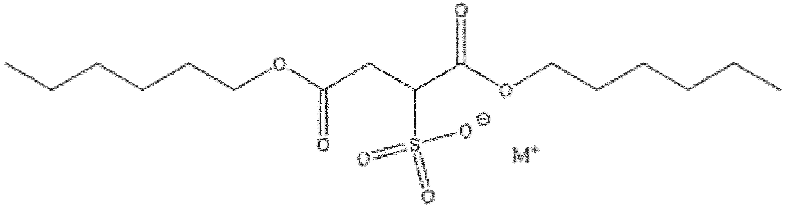 worin M+ Na+, K+, NH4+, (HOCH2CH2)3NH+ bedeutet, oder
- Ethyl-2-hexylsulfosuccinat der Formel: 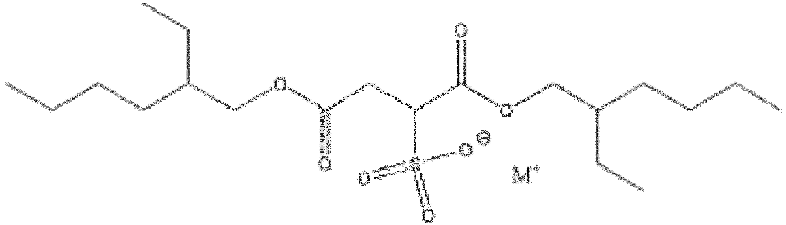 worin M⁺ Na⁺, K⁺, NH₄⁺, (HOCH₂CH₂)₃NH⁺ bedeutet,
• Alkylarylsulfonaten der Formel: 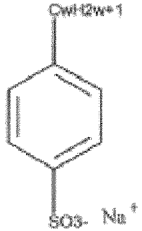 worin w eine ganze Zahl zwischen 8 und 12 ist,
und insbesondere Natriumisooctylbenzensulfonat, Natriumisononylbenzensulfonat oder Natriumisododecylbenzensulfonat der Formel: 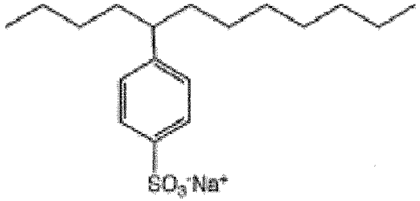
• Propoxysulfaten der Formel: 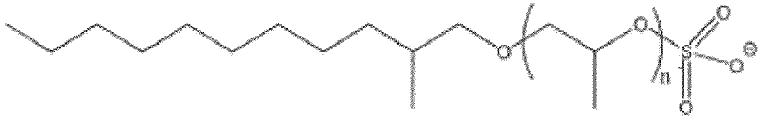 worin die Anzahl der Propoxylat-Einheiten n von 4 bis 8 beträgt
• Alkylsulfaten, insbesondere Salze von Laurylsulfat, wie Natriumlaurylsulfat, auch bekannt als Natriumdodecylsulfat (SDS), Ammoniumlaurylsulfat (ALS); Natriumalkylethersulfaten, wie Natriumlaurylethersulfat (Laurethsulfat) (LES); Natriumcocosulfat (SCS)
• und Salzen von Fettsäuren der Formel R-CO₂⁻ M⁺, worin R eine lineare oder verzweigte, gesättigte oder ungesättigte Kohlenstoffkette mit 8 bis 18 Kohlenstoffatomen darstellt und M⁺ ein Kation darstellt, das ausgewählt ist aus den Ionen Na⁺, K⁺, NH₄⁺, (HOCH₂CH₂)₃NH⁺, insbesondere das Salz der Ölsäure der Formel CH₃(CH₂)₇CH=CH(CH₂)7CO₂⁻ M⁺, worin M⁺ die oben definierten Bedeutungen hat.

4. Mikroemulsion nach einem der Ansprüche 1 bis 3, wobei das Alkylglucosid insbesondere ausgewählt ist aus Heptylglucosid, Octylglucosid, Decylglucosid und Mischungen davon, und vorzugsweise Heptylglucosid ist.

5. Mikroemulsion nach einem der Ansprüche 1 bis 4, wobei:
- die Menge an anionischem Tensid kleiner oder gleich 2 Gew.-%, bezogen auf das Gesamtgewicht der Mikroemulsion, ist,
- die Menge an Alkylglucosid von 0,5 bis 13 Gew.-%, bezogen auf das Gesamtgewicht der Mikroemulsion, und vorzugsweise von 0,5 bis 8 Gew.-% beträgt.

6. Mikroemulsion nach einem der Ansprüche 1 bis 5, wobei das Verhältnis von anionischem Tensid : Alkylglucosid 1 : 4 bis 1 : 12, vorzugsweise 1 : 4 bis 1 : 10 beträgt.

7. Mikroemulsion nach einem der Ansprüche 1 bis 6, wobei die Menge an Lösungsmitteltensid von 4 bis 10 Gew.-% beträgt, bezogen auf das Gesamtgewicht der Mikroemulsion.

8. Mikroemulsion nach einem der Ansprüche 1 bis 7, die im wesentlichen frei von Ethanol ist, vorzugsweise frei von Ethanol.

9. Verwendung einer Mikroemulsion nach einem der Ansprüche 1 bis 8 zur Herstellung einer Feinparfümerie-Zusammensetzung oder einer kosmetischen oder Körperpflege-Zusammensetzung.

## Claims

1. A microemulsion of oil-in-water type comprising by weight relative to the total weight of microemulsion:
• 70% to 94%, preferably 70% to 90%, of water,
• 1% to 15%, preferably 5% to 12%, of at least one hydrophobic fragrancing substance,
• 4% to 20%, preferably 4% to 18%, of at least one preferably volatile solvo-surfactant, which is a monoalkylated glycerol derivative of following formula (I): 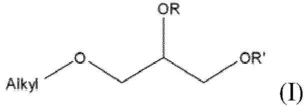 wherein the "alkyl" group is a linear alkyl group comprising from 1 to 5 carbon atoms, and R and R' are each independently H or a linear or branched alkyl group comprising from 1 to 5 carbon atoms, preferably a methyl or ethyl group, with the proviso that R is different from R',
• 0.1% to 15%, preferably 1% to 13%, of at least one anionic surfactant and of at least one alkyl glucoside as hydrotropic agent.

2. The microemulsion as claimed in claim 1, wherein the hydrophobic fragrancing substance is a natural hydrophobic fragrancing substance selected from terpenes, essential oils and natural compounds having odoriferous properties, especially selected from aldehydes, esters, ketones, alcohols, phenols, alkenes and ethers.

3. The microemulsion as claimed in any one of claims 1 to 4, wherein the anionic surfactant is selected from:
• alkyl sulfonates, and in particular:
- sodium C14-17 sulfonate (SAS),
- dihexyl sulfosuccinate (DHS) of formula: 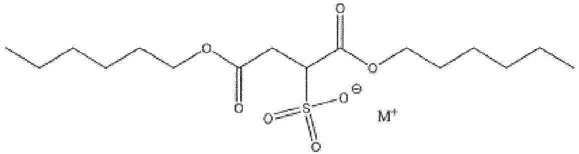 wherein M+ represents Na⁺, K⁺, NH₄⁺, (HOCH₂CH₂)₃NH⁺, or
- 2-ethylhexyl sulfosuccinate of formula: 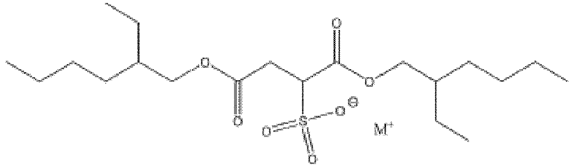 wherein M⁺ represents Na⁺, K⁺, NH₄⁺, (HOCH₂CH₂)ₕNH⁺,
• alkylaryl sulfonates of formula: 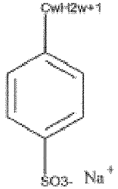 wherein w is an integer from 8 to 12,
and in particular sodium isooctylbenzenesulfonate, sodium isononylbenzenesulfonate or sodium isododecylbenzenesulfonate of formula: 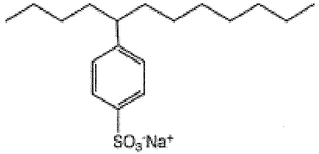
• propoxy sulfates of formula: 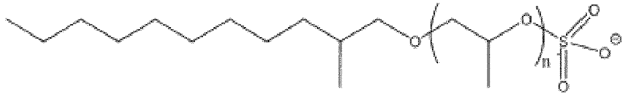 wherein the number of propoxylate units n is from 4 to 8,
• alkyl sulfates, especially salts of lauryl sulfate such as sodium lauryl sulfate also known as sodium dodecyl sulfate (SDS), ammonium lauryl sulfate (ALS); sodium alkylether sulfates such as sodium lauryl ether (laureth) sulfate (LES); sodium coco sulfate (SCS),
• and salts of fatty acids of formula R-CO₂⁻ M⁺, wherein R represents a linear or branched, saturated or unsaturated carbon-based chain containing 8 to 18 carbon atoms, and M⁺ represents a cation selected from the ions Na⁺, K⁺, NH₄⁺, (HOCH₂CH₂)₃NH⁺, especially the oleic acid salt of formula CH₃(CH₂)₇CH=CH(CH₂)₇CO₂⁻ M⁺, wherein M⁺ has the above-defined meanings.

4. The microemulsion as claimed in any one of claims 1 to 3, wherein the alkyl glucoside is in particular selected from heptyl glucoside, octyl glucoside, decyl glucoside and mixtures thereof, and is preferably heptyl glucoside.

5. The microemulsion as claimed in any one of claims 1 to 4, wherein:
- the amount of anionic surfactant is less than or equal to 2% by weight relative to the total weight of microemulsion,
- the amount of alkyl glucoside is from 0.5% to 13% by weight relative to the total weight of microemulsion, and preferably from 0.5% to 8%.

6. The microemulsion as claimed in any one of claims 1 to 5, wherein the anionic surfactant: alkyl glucoside ratio is from 1:4 to 1:12, and preferably from 1:4 to 1:10.

7. The microemulsion as claimed in any one of claims 1 to 6, wherein the amount of solvo-surfactant is from 4% to 10% by weight relative to the total weight of microemulsion.

8. The microemulsion as claimed in any one of claims 1 to 7, which is substantially free of ethanol, preferably which is devoid of ethanol.

9. The use of a microemulsion as claimed in any one of claims 1 to 8 for the preparation of a fine fragrance composition or of a cosmetic or body hygiene composition.
